(19) Europäisches Patentamt
European Patent Office
Office européen des brevets

(11) Numéro de publication: **0 008 249**
**B1**

(12) **FASCICULE DE BREVET EUROPEEN**

(45) Date de publication du fascicule de brevet: **26.08.81**

(21) Numéro de dépôt: **79400459.8**

(22) Date de dépôt: **05.07.79**

(51) Int. Cl.³: **C 07 D 471/16,**
A 61 K 31/505,
A 61 K 31/55,
C 07 D 471/14
//C07D471/04, (C07D471/16,
243/00, 221/00, 209/00),
(C07D471/16, 239/00,
221/00, 209/00),
(C07D471/14, 239/00,
221/00, 209/00),
(C07D471/14, 235/00,
221/00, 209/00)

(54) **Dérivés de fluorènes et fluoranthènes, leur procédé de préparation et leur application en thérapeutique.**

(30) Priorité: **28.07.78 FR 7822352**
**28.07.78 FR 7822353**

(43) Date de publication de la demande:
**20.02.80 Bulletin 80/4**

(45) Mention de la délivrance du brevet:
**26.08.81 Bulletin 81/34**

(84) Etats Contractants Désignés:
**AT BE CH DE FR GB IT LU NL SE**

(56) Documents cités:
**CH - A - 601 299**

(73) Titulaire: **SYNTHELABO**
**1, avenue de Villars**
**F-75341 Paris Cedex 07 (FR)**

(72) Inventeur: **Dupont, Régis**
**141 Bld de la gare**
**F-75013 Paris (FR)**
Inventeur: **Lardenois, Patrick André Louis**
**18, rue Varengue**
**F-92340 Bourg-la-Reine (FR)**
Inventeur: **Morel, Claude Constant Henri**
**15, rue de la Bergerie**
**F-91300 Massy (FR)**
Inventeur: **Frost, Jonathan Reid**
**122 Résidence Les Eaux-Vives**
**F-91120 Palaiseau (FR)**
Inventeur: **Koletar, Gabor Itsvan**
**212 Dogwood Lane**
**Berkeley Heights, New Jersey 07922 (US)**

(74) Mandataire: **Thouret-Lemaitre, Elisabeth**
**Service Brevets - SYNTHELABO 58, rue de la Glacière**
**F-75621 Paris Cedex 13 (FR)**

**EP 0 008 249 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Dérivés de fluorènes et fluoranthènes, leur procédé de préparation et leur application en thérapeutique

La présente invention concerne des dérivés d'octahydrotriazacyclo-hepta-fluorènes et de triaza-fluoranthènes, sous forme de racémates ou d'énantiomères, leurs sels d'addition aux acides pharmaceutiquement acceptables, leur préparation et leur application en thérapeutique.

Les composés de l'invention répondent à la formule (I)

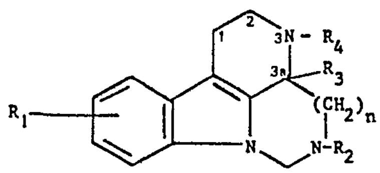

dans laquelle
n est 1 ou 2,
$R_1$ est un atome d'hydrogène ou d'halogène ou un radical $(C_{1-4})$-alkyle ou $(C_{1-4})$-alcoxy,
$R_2$ est un atome d'hydrogène, un radical $(C_{1-4})$-alkyle, cyclopropyle, cyclopropyl-méthyle, benzyle, halogénobenzyle, $(C_{1-4})$-alkyle-benzyle, $(C_{1-4})$-alcoxy-benzyle, acétyle, $(C_{1-4})$-alcoxycarbonyle ou $(C_{1-4})$-alkyl- -aminocarbonyle, $R_3$ est un atome d'hydrogène ou un radical $(C_{1-4})$-alkyle, $R_4$ est un atome d'hydrogène, un radical $(C_{1-4})$-alkyle, acétyle, propanoyle, benzoyle ou butanoyle.

Les composés (I) possèdent un carbone asymétrique en position 3a. Les racémates et les énantiomères des composés (I) font partie de l'invention.

Selon l'invention, on prépare les composés (I) dans lesquels $R_4 = H$ à partir des composés de formule (VII)

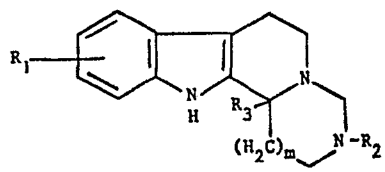

(VII)

dans laquelle $R_1$ et $R_3$ ont les significations données ci-dessus $R_2$ est un radical $(C_{1-4})$-alkyle, cyclopropyle, cyclopropyl-methyle, benzyle, halogènobenzyle, $(C_{1-4})$ alkyl-benzyle ou $(C_{1-4})$-alcoxy-benzyle, et m=0 ou 1, par transposition en milieu acide.

On prépare les composés (I) dans lesquels $R_4$ est différent de H à partir des composés (I) dans lesquels $R_4$ est H (éventuellement sans isoler ces derniers) par alkylation ou acylation directe. Les composés (I) pour lesquels $R_4$ est un acyle peuvent être réduits en composés (I) pour lesquels $R_4$ est un alkyle.

Les composés (I) dans lesquels $R_2$ est H sont obtenus à partir des composés (I) dans lesquels $R_2$ est benzyle par débenzylation.

Les composés (I) dans lesquels $R_2$ est un radical acétyle, $(C_{1-4})$-alkylaminocarbonyle ou $(C_{1-4})$-alcoxycarbonyle sont obtenus à partir des composés (I) correspondants dans lesquels $R_2$ est le radical benzyle, par débenzylation effectuée en présence de palladium sur charbon sous une pression de 3 à 4 $kg/cm^2$ d'hydrogène; puis par réaction du composé obtenu avec un composé porteur de groupe $R_2$ (chloroformiate d'alkyle, anhydride d'acide, chlorure d'acide, isocyanate d'alkyle).

Les composés de départ (VII), sous la forme de racémates ou d'énantiomères, sont nouveaux, à l'exception de ceux pour lesquels $m = 1$,
$R_1 = H$ ou Br-9, $R_2 = CH_3$ et $R_3 = H$.

Les composés (VII) pour lesquels $m = 0$

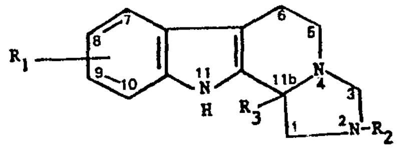

possèdent un carbone asymétrique en position 11b.

Les composés (VII) dans lesquels m = 1

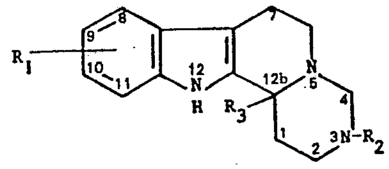

possèdent un carbone asymétrique en position 12b.

On prépare les composés (VII) à partir d'un composé de formule (II)

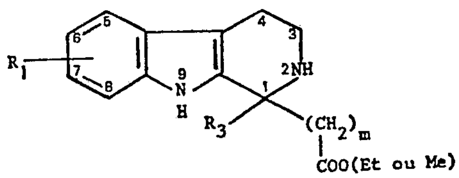

dans laquelle

$R_1$, $R_3$ et m ont les mêmes significations que ci-dessus.

Deux procédés permettent à partir de ces produits de départ (II) de préparer les composés (VII): ils sont représentés dans les schémas 1 et 2 suivants. Dans ces schémas $R_2$ est un radical $(C_{1-4})$-alkyle, cyclopropyle, cyclopropylméthyle, benzyle halogenobenzyle, $(C_{1-4})$-alkylbenzyle, $(C_{1-4})$-alcoxy-benzyle.

Schéma 1

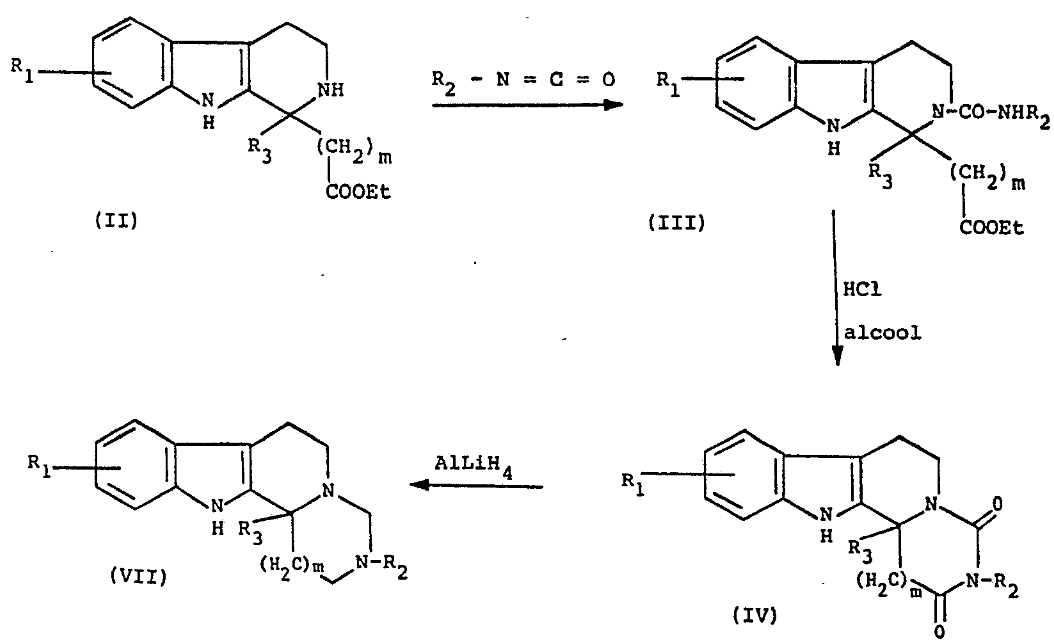


(II) (III) (IV) (VII)

Schéma 2

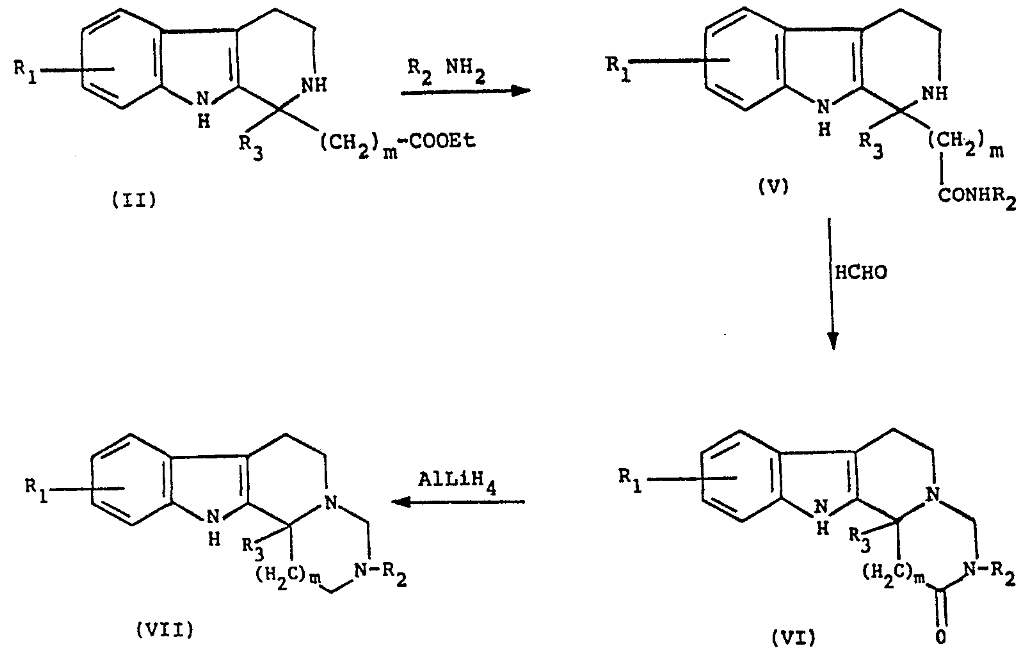

(II) (V) (VI) (VII)

Selon le procédé du schéma 1, on prépare les composés (VII) à partir des composés (II) sous forme de base, par réaction avec un isocyanate de formule $R_2—N=C=O$ dans un solvant neutre tel que le cyclohexane ou le chlorure de méthylène, à une température variant entre la température ambiante et la température d'ébullition du solvant, puis on cyclise le composé (III) obtenu à l'aide d'acide chlorhydrique en milieu alcoolique, à la température d'ébullition de l'alcool, et enfin on réduit le composé (IV) obtenu en composé (VII) à l'aide d'hydrure double de lithium et d'aluminium, dans un solvant tel que le dioxanne ou le tétrahydrofuranne, à la température d'ébullition du solvant.

Lorsque $R_2 = H$, on fait réagir le composé de départ (II) sous forme de sel (par exemple de chlorhydrate) avec de l'isocyanate de sodium dans de l'eau à 50°C.

Les composés (IV) obtenus dans lesquels $R_2$ est H, peuvent alors conduire aux composés (IV) dans lesquels $R_2$ est autre que H par substitution directe sur l'azote (alkylation....).

Selon le procédé du schéma 2, on fait réagir le composé (II) avec une amine de formule $R_2NH_2$, qui sert elle-même de solvant ou dans un solvant tel qu'un alcool, à une température allant de la température ambiante à 100°C, puis on cyclise le composé (V) obtenu à l'aide de formaldéhyde en solution à 30% dans de l'eau et enfin on réduit le composé (VI) à l'aide d'hydrure double de lithium et d'aluminium, dans un solvant tel que le dioxanne ou le tétrahydrofuranne, à la température d'ébullition du solvant.

Les composés de départ (II) sont obtenus de la manière suivante:

Les composés de départ (II) dans lesquels $R_3 =$ alkyle sont préparés à partir de la tryptamine substituée on non selon le schéma suivant:

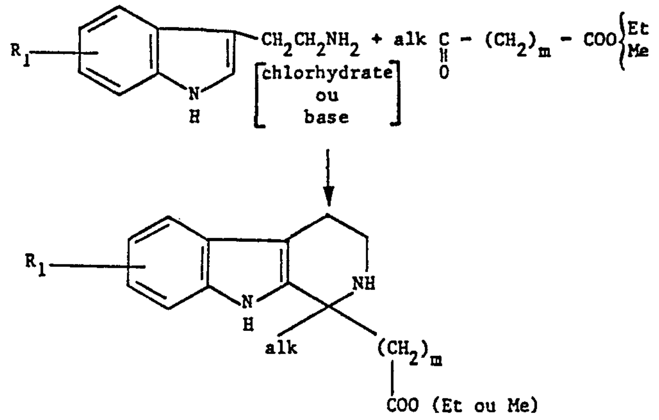


Cette réaction est décrite dans la littérature. Il est possible d'obtenir les composés de départ (II) dans

lesquels $R_3 = H$ et $m = 1$ à partir du chlorohydrate de tryptamine substituée ou non, selon le schéma suivant:

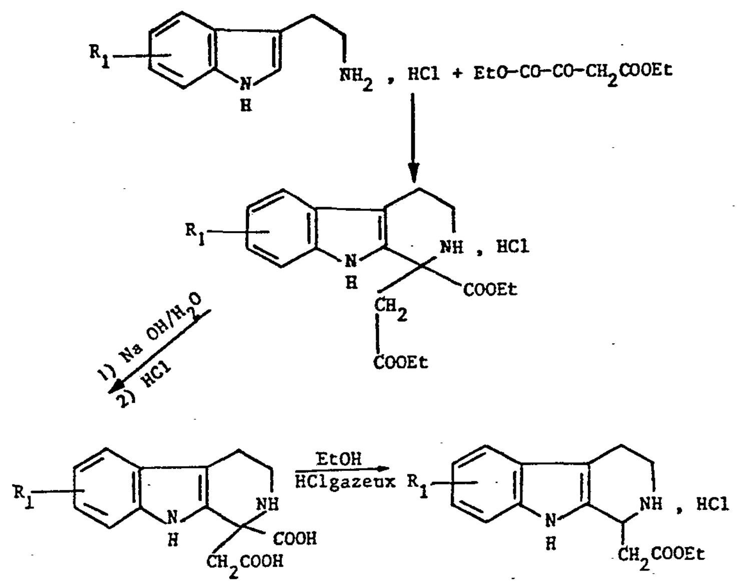


La préparation est la suivante: $R_1 = H$.

1. On prépare une suspension de 115 g (0,55 mole) d'oxalacétate de diéthyl sodium dans 500 $cm^3$ d'EtOH. On y ajoute lentement 50 $cm^3$ de HCl de densité 1,18 (soit 0,55 mole). La suspension obtenue est ajoutée à raison du 1/3 du volume à une suspension de 98,2 g (0,5 mole) de chlorhydrate de tryptamine dans 500 $cm^3$ d'EtOH à l'ébullition. Le reflux est maintenu sous agitation pendant 2 heures puis on introduit le 2ème 1/3 de la suspension, puis, après encore 2 heures de reflux, le dernier 1/3. On distille environ 700 $cm^3$ d'EtOH et laisse refroidir une nuit. On essore la masse cristalline qu'on lave à l'eau (4 × 100 $cm^3$) pour éliminer NaCl puis à l'acétone.

Après séchage on obtient le produit qui fond en se décomposant à 230°C.

2. Une suspension de 67,8 g du diester obtenu précédemment dans 700 $cm^3$ d'eau contenant 70 g de soude est portée à ébullition 48h. Après refroidissement on ajoute 200 $cm^3$ de HCl (d = 1,19). Il se forme un précipité qu'on laisse sous agitation 1 heure. On essore, lave plusieurs fois à l'eau, puis à l'acétone et enfin à l'éther.

3. 25 g du diacide précédent sont mis en suspension dans 180 $cm^3$ d'EtOH. Sous agitation on sature en HCl gazeux. L'échauffement s'accompagne d'une dissolution totale. On chauffe pour maintenir le reflux pendant une nuit. Après refroidissement on essore, lave avec EtOH puis l'éther. On obtient des cristaux blancs de tétrahydro-2,3,4,9 1H-pyrido [3,4-b] indole - 1 - acétate d'éthyle sous forme de chlorhydrate.

F = 260°C.

Le composé de départ (II) dans lequel $R_3 = H$ et $m = 0$ lorsque $R_1 = H$ est décrit dans la littérature (Z. J. Vejdélek et collaborateurs, J. of Med. and Pharm. Chem. Vol. 3 n° 3 (1961) p. 427—440).

Les exemples suivants illustrent l'invention. Les micro-analyses et les spectres IR et RMN confirment la structure des composés.

Exemple 1

*Méthyl-6 octahydro-1,2,3,3a,4,5,6,7 triaza-3,6,7a cyclohepta [j k] fluorène*

$[n = 2 \; R_1 = H \; R_2 = CH_3 \; R_3 = H \; R_4 = H]$

1. Carbamoyl-2 tétrahydro-2,3,4,9 pyrido [3,4-b] indole-1-acétate d'éthyle.

On dissout 26 g (0,088 mole) de chlorhydrate de tétrahydro - 2,3,4,9 - 1H pyrido [3,4b] indole - 1 - acétate d'éthyle (composé de départ II) dans 1 1 d'eau chaude. Le pH doit être de 4—5. Lorsque le milieu réactionnel est à 50°C, on ajoute une solution de 8,7 g (0,13 mole) de cyanate de sodium dans 150 $cm^3$ d'eau. Le milieu réactionnel est maintenu à 50°C sous agitation. On agite 4h à 50°C et on laisse une nuit au repos à la température ambiante l'eau surnageante et sous-tirée. On ajoute 200 $cm^3$ d'éther et agite, puis on sous-tire la solution éthérée, et recommence la même opération. Le produit attendu est très soluble dans l'éther. Les extraits éthérés sont réunis, lavés à l'eau acidulée afin d'éliminer les traces du produit de départ, puis à l'eau.

On sèche sur $Na_2SO_4$ et évapore le solvant.

On obtient une huile qui évolue en mousse par dessication plus poussée. Le produit est utilisé tel quel dans le stade suivant.

2. Octahydro-1,2,3,4,6,7,12,12b-pyrimido [1',6':1,2] pyrido [3,4-b] indole-dione-2,4.

On dissout 11,1 g du composé précédent dans 250 $cm^3$ d'éthanol à reflux. A cette température on ajoute une solution saturée de HCl gazeux dans 55 $cm^3$ d'éthanol. On maintient ce mélange à la temperature de reflux pendant 5 h puis on laisse une nuit au repos à la température ambiante. Le produit est essoré, lavé avec EtOH puis à l'éther. On sèche. On obtient un produit très insoluble dans les solvants organiques. Après recristallisation dans du diméthylformamide, il fond à 365°C.

3. Méthyl-3 octahydro-1,2,3,4,6,7,12,12b-pyrimido [1', 6': 1,2] pyrido [3,4-b] indole-dione-2,4

A une solution de 8 g (0,031 mole) du composé précédent dans 300 $cm^3$ de diméthylformamide, on ajoute 1,5 g (0,031 mole) d'hydrure de sodium en dispersion à 50% dans de l'huile. On agite pendant 1h30 à l'abri de l'humidité puis on ajoute 4,5 g (0,031 mole) d'iodure de méthyle et on laisse le milieu réactionnel une nuit à la température ambiante tout en agitant.

Le résidu minéral est éliminé par filtration puis le filtrat est évaporé. On reprend le résidu à l'eau, ce qui provoque sa concrétisation en solide blanc. Celui-ci est recristallisé dans de l'éthanol. Le composé pur, obtenu après recristallisation dans de la méthyl-éthyl-cétone, fond à 246—248°C.

4. Méthyl-3 octahydro-1,2,3,4,6,7,12,12b-pyrimido [1', 6' : 1,2]pyrido-[3,4-b] indole.

A une suspension bouillante de 4 g de $LiAlH_4$ dans 80 $cm^3$ de dioxanne, on ajoute goutte à goute, en 30 mn, une suspension de 4 g du composé obtenu sous 3 dans 80 $cm^3$ de dioxanne. Le reflux est maintenu 1 h après la fin de l'addition. Après refroidissement l'excès d'hydrure est detruit par un peu d'eau. On filtre pour éliminer le précipité minéral et on lave ce dernier plusieurs fois à l'éther. Le filtrat organique est séché sur $Na_2SO_4$ puis évaporé. L'huile obtenue est purifiée par passage sur colonne de gel de silice (100 g) à l'aide d'un mélange chloroforme/méthanol 4/1. Par trituration dans de l'acétate d'éthyle on obtient le composé qui après recristallisation fond à 168°C.

5. Méthyl-6 octahydro-1,2,3,3a,4,5,6,7 triaza-3,6,7a-cyclohepta [j k] fluorène

On agite une solution de 11,1 g de méthyl-3 octahydro-1,2,3,4,6,7,12,12b-pyrimido [1', 6' : 1, 2] pyrido[3, 4-b] indole dans 100 $cm^3$ de méthanol et on ajoute 15 $cm^3$ de méthanol saturé d'acide chlorhydrique. On agite la solution pendant une nuit à la température du laboratoire. On dilue par de l'éther, essore, met le solide en présence de $NH_4OH$, ajoute de l'acétate d'éthyle, décante la phase organique, lave à l'eau et sèche sur $Na_2SO_4$. On évapore le solvant. On obtient un résidu solide jaune qu'on triture dans de l'éther et qu'on essore.

F = 108—109°C (éther de pétrole).

### Exemple 2

*Acétyl-3 méthyl-6 octahydro-1,2,3,3a,4,5,6,7 triaza-3,6,7a cyclohepta [j k] fluorène.*

[n = 2 $R_1$ = H $R_2$ = $CH_3$ $R_3$ = H $R_4$ = $CH_3CO$]

On alcalinise 5 g de chlorohydrate de méthyl-6 octahydro-1,2,3,3a,4,5,6,7 triaza-3,6,7a cyclohepta [j k] fluorène (composé I, n = 2, $R_2$ = $CH_3$, $R_1$ = $R_3$ = $R_4$ = H) avec du $NaHCO_3$ et on extrait la base libre dans du $CHCl_3$. On évapore les extraits chloroformiques, on dissout le solide obtenu dans 10 ml de pyridine et on ajoute 6 ml d'anhydride acétique. On agite le mélange réactionnel 15 mm à la température ambiante puis on chauffe à la température de reflux pour achever la réaction. On verse le mélange réactionnel refroidi dans de l'eau (100 ml). On extrait avec du chloroforme.

Après évaporation des extraits chloroformiques on obtient une huile qui triturée dans de l'éthanol fournit un solide. Après recristallisation dans de l'éthanol le produit fond à 184°C.

### Exemple 3

*Acétyl-3 méthoxy-10 méthyl-6 octahydro-1,2,3,3a,4,5,6,7 triaza-3,6,7a cyclohepta [j k] fluorène*

[n = 2 $R_1$ = $CH_3O$-10 $R_2$ = $CH_3$ $R_3$ = H $R_4$ = $CH_3CO$]

1. 1H-pyrido [3,4-b] méthyl-6 tétrahydro-2,3,4,9 indole-1 acétate d'éthyle.

On introduit dans un ballon 20 g de chlorhydrate de méthoxy-5 tryptamine et 100 ml d'éthanol et on porte à la température de reflux. On place dans un erlenmeyer 20,3 g d'oxalacétate de diéthyl sodium et 100 ml d'éthanol. On ajoute 8 ml d'acide chlorohydrique concentré et on agite pendant 10 mn. On introduit la suspension de l'oxalate dans l'éthanol dans la suspension de la tryptamine et on porte à la température de reflux pendant 7h. On laisse refroidir une nuit. On chasse le solvant et alcalinise le résidu avec de l'ammoniaque. On extrait 3 fois avec 300 ml de chloroforme. On chasse le

chloroforme et obtient une huile que l'on chromatographie sur silice. Après élution avec un mélange $EtOH/CHCl_3$ (2/8) on obtient une huile quie cristallise avec l'éther de pétrole. Après recristallisation le produit fond à 100°C.

On ajoute 150 ml d'une solution à 10% NaOH et on chauffe à la température de reflux pendant 20h. On acidifie avec 50 ml d'acide chlorhydrique concentré et on essore. On ajoute au composé obtenu un mélange d'éthanol (250 ml) et d'acide sulfurique concentré (20 ml). On porte à la température de reflux pendant 4h. Après refroidissement on alcalinise le résidu et on extrait avec du chloroforme. Après évaporation du solvant et chromatographie de l'huile obtenue sur silice, on obtient un composé dont on prépare le maléate dans de l'éthanol.

F = 181°C.

2. N-méthyl-[méthoxy-6 tétrahydro-2,3,4,9 1H-pyrido [3-4b] indole]-acétamide-1.

On place dans une bombe 15 g du composé obtenu sous 1, (base) et on ajoute 500 ml d'une solution de méthylamine à 33% dans de l'éthanol. On chauffe à 100°C pendant 7h. On laisse refroidir une nuit. On chasse l'éthanol et on obtient un solide brun que l'on fait cristalliser dans de l'éthanol. F = 190°C.

3. Méthoxy-9 méthyl-3, octahydro-1,2,3,4,6,7,12,12b oxo-2 pyrimido [1', 6' : 1,2] pyrido [3, 4b] indole.

On place dans un erlenmeyer 10,3 g du composé obtenu sous 2. On ajoute 100 ml d'éthanol et 10 ml d'une solution de formaldehyde à 30% dans de l'eau. On agite pendant 2h. On chasse le solvant et on obtient un solide blanc que l'on fait recristalliser dans de l'éthanol.

F = 224°C.

4. Méthoxy-9 méthyl-3 octahydro-1,2,3,4,6,7,12,12b pyrimido [1', 6' : 1,2] pyrido [3,4-b] indole.

On introduit dans un ballon 4 g de $LiAlH_4$ et 100 ml de THF. On porte à la température de reflux et on ajoute 7,5 g du composé obtenu sous 3 en solution dans 100 ml de THF, en 10 mn. On maintenant à la température de reflux pendant 1h. On laisse refroidir et ajoute de l'eau et de la soude. On filtre et chasse le solvant. Le solide blanc recristallisé dans de l'acétate d'éthyle, fond à 199—200°C.

5. Acétyl-3 méthoxy-10 méthyl-6 octahydro-1,2,3,3a,4,5,6,7 triaza-3,6,7a cyclohepta [j k] fluorène.

On transpose en milieu acide le composé précédent. On ajoute 20 ml de pyridine et 3,3 ml d'anhydride acétique au composé obtenu. On agite à la température ambiante pendant 1 h. On chasse la pyridine et obtient un solide que l'on fait recristalliser dans de l'éthanol.

F = 165°C.

## Exemple 4

*Ethyl-3 méthyl-6 octahydro-1,2,3,3a,4,5,6,7 triaza-3,6,7a cyclohepta [j k] fluorène*

$[n = 2 \ R_1 = H \ R_2 = CH_3 \ R_3 = H \ R_4 = C_2H_5]$

On agite 2,5 g d'acétyl-3 méthyl-6 octahydro-1,2,3,3a,4,5,6,7 triaza-3,6,7a cyclohepta [j k] fluorène (composé I, $R_1 = H$, $R_2 = CH_3$, $R_3 = H$, $R_4 = CH_3CO$, $n = 2$) avec 2,4 g de $LiAlH_4$ dans 60 ml de dioxanne, à la température ambiante, pendant 10 mn. On porte lentement le mélange à la température de reflux et l'y maintient pendant 1 heure. On refroidit le mélange réactionnel et détruit l'excès de $LiAlH_4$. On élimine le précipité minéral par filtration puis évapore le filtrat. On obtient une huile que l'on purifie par passage sur une colonne de $SiO_2$. On élue avec un mélange $EtOH/CHCl_3$ 2/8. Après recristallisation dans de l'éther de pétrole, le produit fond à 78—79°C.

## Exemple 5

*Benzyl-3 octahydro-1,2,3,3a,4,5,6,7-triaza-3,6,7a cyclohepta [j k] fluorène*

$[n = 2 \ R_1 = H \ R_2 = C_6H_5CH_2 \ R_3 = H \ R_4 = H]$

1. N-benzyl-[tétrahydro-2,3,4,9 1H-pyrido[3, 4-b]indole]-acétamide-1.

On libère la base de 60 g (0,20 mole) du chlorhydrate de tétrahydro-2,3,4,9 1H-pyrido [3, 4-b] indole-1-acétate d'éthyle dans un mélange eau-ammoniaque/chlorure de méthylène. Après séparation des phases, séchage de la phase organique sur $Na_2SO_4$, le solvant est évaporé. La gomme résultante est dissoute dans 200 $cm^3$ de benzylamine. On chauffe cette solution 42 heures à 110—120°C sous agitation. La benzylamine est éliminée à cette température par distillation sous vide. Après refroidissement il y a amorce de cristallisation puis prise en masse. On triture dans de l'éther, essore les cristaux

et les lave à l'éther. On obtient le composé qui après recristallisation dans de l'acétate d'éthyle fond à 160°C.

2. Benzyl-3 octahydro-1,2,3,4,6,7,12,12b-oxo-2 pyrimido [1', 6' : 1, 2]pyrido[3, 4-b] indole.

On fait tiédir une suspension de 43,4 g (0,135 mole) du composé précedent dans 650 $cm^3$ d'éthanol jusqu'à dissolution. On ajoute 40 $cm^3$ de formaldehyde en solution a 30% dans de l'eau et on agite à 40°C pendant 2 heures, puis une nuit à température ambiante. L'évaporation du solvant fournit une pâte qui concrétise lentement. Par trituration dans de l'ether on obtient des critaux fins, qu'on essore, lave à l'éther et sèche. Après recristallisation dans de l'éthanol le composé fond à 198—200°C.

3. Benzyl-3 octahydro-1,2,3,4,6,7,12,12b-pyrimido [1', 6' : 1, 2]pyrido [3, 4-b] indole.

A une suspension bouillante de 12 g (0,3 mole) de $LiAlH_4$ dans 120 $cm^3$ de tétrahydrofuranne on ajoute, goutte à goutte une solution de 24,2 g (0,073 mole) du composé précédent dans 400 $cm^3$ de tétrahydrofuranne. On maintient le reflux 1h après la fin de l'addition. Après refroidissement l'exces d'hydrure est détruit.

Le résidu minéral est éliminé par filtration et lavé à l'éther. On sèche le fitrat sur $Na_2SO_4$ puis on évapore le solvant. On obtient une masse cristalline blanche qu'on triture dans de l'éther diisopropylique et qu'on essore. Après recristallisation dans de l'acétate d'éthyle le produit fond à 158°C.

4. Benzyl-6 octahydro-1,2,3,3a,4,5,6,7-triaza-3,6,7a cyclohepta [j k] fluorène.

On agite 51,6 g du composé obtenu précédemment sous 3 durant 48 h, à la température ambiante, dans 500 $cm^3$ d'éthanol saturé de HCl gazeux. Le précipité formé est essoré sur fritté, lavé à l'éthanol puis à l'éther. Le produit est séché à l'étuve sous vide à 50°C. On obtient 62,8 g du dichlorohydrate du composé.

La base après libération de son sel et recristallisation dans de l'isopropanol, fond à 129°C.

## Exemple 6

*Acétyl-3 benzyl-6 octahydro-1,2,3,3a,4,5,6,7 triaza-3,6,7a cyclohepta [j k] fluorène*

$$[n = 2 \; R_1 = H \; R_2 = C_6H_5CH_2 \; R_3 = H \; R_4 = CH_3CO]$$

On met en solution 30 g (0,076 mole) de chlorhydrate de benzyl-6 octahydro-1,2,3,3a,4,5,6,7 triaza-3,6,7a cyclohepta [j k] fluorène (composé (I) $n = 2$ $R_1 = H$ $R_2 = C_6H_5CH_2$ $R_3 = R_4 = H$) dans 100 $cm^3$ de pyridine puis on ajoute 30 $cm^3$ d'anhydride acétique. On agite à la température ambiante pendant une nuit. La masse cristalline est essorée. On évapore le filtrat sous vide, puis on reprend le résidu par du dichlorométhane. On dissout la masse cristalline dans du dichlorométhane. Les fractions organiques sont réunies, lavées à l'eau, séchées sur $Na_2SO_4$ puis évaporées sous vide. Le résidu obtenu est débarrassé des traces de pyridine par évaporation sous vide en présence de toluène. Le résidu solide est trituré dans de l'éther puis essoré. Après lavage à l'éther et séchage, on obtient le composé acétylé. Après recristallisation dans de l'acétate d'éthyle, le composé fond à 165—166°C.

## Exemple 7

*Acétyl-3 éthoxycarbonyl-6 octahydro-1,2,3,3a,4,5,6,7 triaza-3,6,7a cyclohepta [j k] fluorène*

$$[n = 2 \; R_1 = H \; R_2 = COOC_2H_5 \; R_3 = H \; R_4 = CH_3CO]$$

1. On soumet à une hydrogénolyse un mélange de 20g du composé obtenu dans l'exemple 6 dans 250 $cm^3$ d'acide acétique et de 2 g de palladium sur charbon à 10%, à la température ambiante, pendant 24h, sous une pression de 3,5 $kg/cm^2$ d'hydrogène. On filtre pour éliminer le catalyseur. Le filtrat est évaporé sous vide puis dilué à l'eau, neutralisé par de l'ammoniaque diluée puis extrait par de l'acétate d'éthyle. Après séchage sur $Na_2SO_4$, on évapore le solvant. Le résidu huileux est dissous dans 20 $cm^3$ de chloroforme et est abandonné au réfrigérateur pendant 48h. On essore le composé obtenu. Après recristallisation dans l'acétate d'éthyle, il fond à 138°—140°C.

2. On met en suspension 10 g de ce composé dans un mélange de 150 $cm^3$ d'éther et de 150 $cm^3$ d'eau contenant 2,3 g de potasse en pastilles. Tout en agitant on ajoute 3,2 $cm^3$ de chloroformiate d'éthyle. On agite 5h. On ajoute de l'acétate d'éthyle jusqu'à dissolution totale du précipité. La phase organique est séparée, lavée à l'eau, séchée sur $Na_2SO_4$ puis évaporée. Le solide blanc obtenu est trituré dans de l'éther puis essoré et séché. Après recristallisation dans de l'éthanol, le composé fond à 195—196°C.

Exemple 8

*Acétyl-3 méthyl-3a méthyl-5 fluoro-9 hexahydro-2,3,3a,4,5,6 triaza-3,5,6a fluoranthène*

[n = 1 $R_1$ = F-9 $R_2 = CH_3$ $R_3 = CH_3$ $R_4 = CH_3CO$]

1. Méthylaminocarbonyl-1 méthyl-1 fluoro-9 tétrahydro-2,3,4,9 1H-pyrido [3, 4-b] indole.

— on met en suspension 30 g (0,139 mole) de chlorhydrate de fluoro-5 tryptamine dans 450 ml de méthanol. On ajoute 15 ml de pyruvate de méthyle. On agite à la température ambiante pendant 5j. On chasse le solvant au rotavapor. On reprend le résidu par un mélange/eau/acétate d'éthyle.

On alcalinise avec une solution N de soude. La phase organique est décantée, lavée et séchée puis évaporée. Le résidu cristallisé est repris par 20 ml de $CH_2Cl_2$. On agite 15 mn dans un bain de glace et on filtre le précipité. On le recristallise dans du toluène. F = 177°C.

— on introduit dans un ballon 3,2 g (0,0122 mole) du composé obtenu précédemment et 50 ml d'une solution à 33% de méthylamine dans de l'éthanol. On agite à la température ambiante pendant 24h. On filtre le précipité que l'on fait recristalliser dans du n-propanol. F = 238°C.

2. Méthyl-2 méthyl-11b fluoro-8 hexahydro-2,3,5,6,11,11b oxo-1 imidazo [1', 5' : 1,2] pyrido [3,4-b] indole.

On introduit 5,4 g (0,0206 mole) du composé obtenu sous 1 dans 100 ml d'éthanol. On ajoute 1,5 g de potasse en pastilles puis 15 ml d'une solution à 30% d'aldehyde formique. On chauffe au bain d'huile à 70°C pendant 24h. On réduit la solution éthanolique à 20 ml et filtre le précipité. Après recristallisation dans l'éthanol il fond à 223°C.

3. Méthyl-2 méthyl-11b fluoro-8 hexahydro-2,3,5,6,11,11b imidazo [1', 5' : 1,2] pyrido [3, 4b] indole.

On ajoute 2,85 g (0,0104 mole) du composé obtenu sous 2 en solution dans du tétrahydrofuranne anhydre (40 ml) à une suspension de 1 g de $LiAlH_4$ dans 25 ml de THF anhydre. On chauffe à la température de reflux pendant 2h. On refroidit et on détruit l'excès d'hydrure. Après évaporation du solvant d'extraction on obtient un résidu cristallisé. F = 203°C.

4. Acétyl-3 méthyl-3a méthyl-5 fluoro-9 hexahydro-2,3,3a,4,5,6, triaza-3,5,6a fluoroanthène.

On traite 2,65 g (0,102 mole) du composé obtenu sous 3 par 30 ml d'une solution de nitrométhane à 10% d'acide sulfurique. On agite à la température ambiante pendant 48h. On chasse le solvant au rotavapor. On reprend le résidu par de la glace. On ajoute de l'acétate d'éthyle et on alcalinise avec de l'ammoniaque. On décante la phase organique et évapore le solvant. On reprend le résidu par 25 ml de pyridine et on ajoute 1 ml d'anhydride acétique. On agite à la température ambiante pendant 2h. On évapore la solution au bain-marie sous vide. On reprend le résidu par de la glace. On traite par une solution aqueuse N de NaOH et extrait à l'acétate d'éthyle. La phase organique lavée et séchée est evaporée. On fait recristalliser le résidu dans de l'oxyde d'isopropyle.

F = 110°C.

Les composés de l'invention préparés à titre d'exemples sont représentés dans le tableau suivant:

TABLEAU 1

Composés de formule (I)

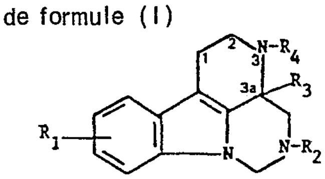

n = 1

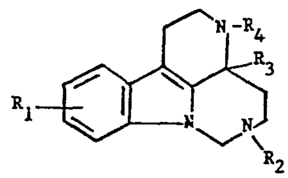

n = 2

| Composé n° | n | $R_1$ | $R_2$ | $R_3$ | $R_4$ | F (°C) |
|---|---|---|---|---|---|---|
| 1 | 2 | H | $CH_3$ | H | H | 108–9 |
| 2 | 2 | H | $C_2H_5$ | H | H | 240–2 (dec) |
| 3 | 2 | H | $nC_3H_7$ | H | H | 109 |
| 4 | 2 | H | $nC_4H_9$ | H | H | 74 |
| 5 | 2 | H | $CH_3$ | H | $CH_3CO$ | 184 |
| 6 | 2 | Cl–10 | $CH_3$ | H | $CH_3CO$ | 151 |
| 7 | 2 | F–10 | $CH_3$ | H | $CH_3CO$ | 188 |
| 8 | 2 | $CH_3$–10 | $CH_3$ | H | $CH_3CO$ | 174 |
| 9 | 2 | H | $CH_3$ | H | $C_2H_5CO$ | 136,5 |
| 10 | 2 | H | 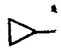 | H | H | 137 |
| 11 | 2 | H | $CH_3$ | H | $C_2H_5$ | 79 |
| 12 | 2 | $CH_3$–10 | $CH_3$ | H | $C_2H_5$ | huile |
| 13 | 2 | C–10 | $CH_3$ | H | $C_2H_5$ | 121 |
| 14 | 2 | H | $CH_3$ | H | $C_6H_5CO$ | 168–9 |
| 15 | 2 | H | $CH_3$ | $CH_3$ | $CH_3CO$ | 142 |
| 16 | 2 | H | $C_6H_5CH_2$ | H | H | 129 |
| 17 | 2 | H | $CH_3$ | H | $C_3H_7$ | huile |
| 18 | 1 | H | $CH_3$ | $CH_3$ | $CH_3CO$ | 145 |
| 19 | 1 | F–9 | $CH_3$ | $CH_3$ | $CH_3CO$ | 110 |
| 20 | 2 | H | 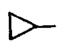 | H | $COCH_3$ | 187–8 |
| 21 | 2 | H | $CH_3$ | H | $COCH_2CH_2CH_3$ | 108 |
| 22 | 2 | H | $C_6H_5CH_2$ | H | $COCH_3$ | 165–6 |
| 23 | 2 | H | H | H | $COCH_3$ | 138–140 |
| 24 | 2 | H | $CH_3$ | H | $CH_2CH_2CH_2CH_3$ | huile |

TABLEAU 1 (Suite)

| Composé n° | n | $R_1$ | $R_2$ | $R_3$ | $R_4$ | F (°C) |
|---|---|---|---|---|---|---|
| 25 | 2 | H | $C_6H_5CH_2$ | H | $CH_2CH_3$ | 100–101 |
| 26 | 2 | H | $C_6H_5CH_2$ | H | $COCH_2CH_3$ | 133–4 |
| 27 | 2 | H | $COCH_3$ | H | $COCH_3$ | 207–8 |
| 28 | 2 | H | $COOC_2H_5$ | H | $COCH_3$ | 195–6 |
| 29 | 2 | $CH_3O$–10 | $CH_3$ | H | $COCH_3$ | 165 |
| 30 | 2 | H | $CONHCH_3$ | H | $COCH_3$ | >300 |

Les composés (VII) intermédiaires sont représentés dans le tableau suivant (II):

TABLEAU II

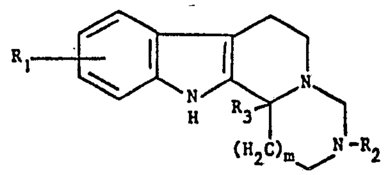

| Composé n° | m | $R_1$ | $R_2$ | $R_3$ | F (°C) |
|---|---|---|---|---|---|
| 1 | 1 | Cl–9 | $CH_3$ | H | 218–9 |
| 2 | 1 | F–9 | $CH_3$ | H | 194 |
| 3 | 1 | $CH_3$–9 | $CH_3$ | H | 215 |
| 4 | 1 | H | $CH_3$ | H | 168 |
| 5 | 1 | H | $C_2H_5$ | H | – |
| 6 | 1 | H | $C_3H_7$ | H | – |
| 7 | 1 | H | $C_4H_9$ | H | – |
| 8 | 1 | H | 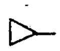 | H | – |
| 9 | 1 | H | $C_6H_5CH_2$ | H | 158 |
| 10 | 1 | H | $CH_3$ | $CH_3$ | 200 |
| 11 | 0 | H | $CH_3$ | H | 209–211 |
| 12 | 0 | H | $CH_3$ | $CH_3$ | 195 |
| 13 | 0 | F–8 | $CH_3$ | $CH_3$ | 203 |
| 14 | 1 | $CH_3O$–9 | $CH_3$ | H | 199–200 |

Les composés de l'invention ont été soumis à des essais pharmacologiques.

Les composés se sont révélés actifs dans le test de l'anoxie hypobare chez la souris et ont une action sur la durée du sommeil induit par le 4-hydroxy-butyrate de sodium (GHB) chez le rat curarisé.

ANOXIE HYPOBARE

Des souris de souche CDL sont maintenues dans une atmosphère appauvrie en oxygène, par réalisation d'un vide partiel (190 mm de mercure correspondant à 5,25% d'oxygène).

Le temps de survie des animaux est noté. Ce temps est augmenté par les agents capables de favoriser l'oxygénation tissulaire et en particulier cérébrale. Les composés étudiés sont administrés, à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100% est déterminée graphiquement.

La DAM des composés de l'invention varie de 10 à 30 mg/kg par voie intrapéritonéale.

ACTION SUR LA DUREE DU "SOMMEIL"

Cette action a été déterminée par l'influence des composés sur la durée du "sommeil" induit par le 4-hydroxybutyrate de sodium (GHB) chez le rat curarisé, sous respiration artificielle dans lequel l'activité électrocorticographique est enregistrée par des électrodes corticales.

Les composés de l'invention diminuent la durée totale du sommeil de −20 à −37%

L'étude pharmacologique des composés de l'invention montre qu'ils sont actifs dans l'épreuve d'anoxie hypobare chez la souris tout en n'étant que peu toxiques et qu'ils exercent une action significative éveillante dans le test du "sommeil" induit par le 4-hydroxy-butyrate de sodium.

Les composés de l'invention, possédant à la fois une activité antianoxique et une activité psychotrope, peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, et la traitement des états dépressifs.

L'invention comprend par conséquent, toutes compositions pharmaceutiques renfermant les composés et/ou leurs sels comme principes actifs, en association avec tous excipients appropriés à leur administration, en particulier par voie orale ou parentérale.

Les voies d'administration peuvent être les voies orale ou parentérale.

La posologie quotidienne peut aller de 10 à 1000 mg.

**Revendications pour les Etats contractants: BE, CH, DE, FR, GB, IT, LU, NL, SE**

1. Dérivés de fluorènes et fluoroanthénes, sous la forme de racémates ou d'énantiomères, répondant à la formule (I)

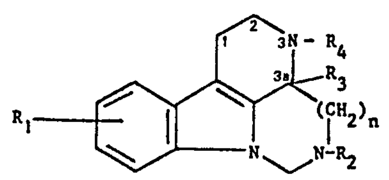

dans laquelle

n est 1 ou 2,

$R_1$ est un atome d'hydrogène ou d'halogène ou un radical $(C_{1-4})$-alkyle ou $(C_{1-4})$-alcoxy,

$R_2$ est un atome d'hydrogène, un radical $(C_{1-4})$-alkyle, cyclopropyle, cyclopropyl -méthyle, benzyle, halogénobenzyle, $C_{1-4})$-alkyl-benzyle, $(C_{1-4})$-alcoxy-benzyle, acétyle, $(C_{1-4})$-alcoxycarbonyle ou $(C_{1-4})$-alkyl-aminocarbonyle,

$R_3$ est un atome d'hydrogène ou un radical $(C_{1-4})$-alkyle,

$R_4$ est un atome d'hydrogène, un radical $(C_{1-4})$-alkyle, acétyle, propanoyle, benzoyle ou butanoyle.

2. Dérivés selon la revendication 1, dans lesquels n est 1 ou 2, $R_1$ est un atome de fluor ou de chlore, le radical méthyle ou méthoxy,

$R_2$ est un atome d'hydrogène, un radical alkyle de 1 à 4 atomes de carbone, le radical benzyle, cyclopropyle, acétyle, éthoxycarbonyle ou méthylaminocarbonyle,

$R_3$ est un atome d'hydrogène ou le radical méthyle

3. Dérivés selon la revendication 2 dans lesquels n est 2.

4. Dérivés selon la revendication 1 dans lesquels n est 2,

$R_1$ est H ou $CH_3O$-10,

$R_2$ est $CH_3$, cyclopropyle ou benzyle,

$R_3$ est H et $R_4$ est $CH_3CO$, $C_6H_5CO$, H, $C_2H_5$ ou $C_3H_7$.

5. Dérivé selon la revendication 4, qui est l'acétyl-3 méthyl-6 octahydro-1,2,3,3a,4,5,6,7 triaza-3,6a,7a cyclohepta [j k] fluorène.

6. Dérivé selon la revendication 4, qui est l'acétyl-3 méthoxy-10 méthyl-6 octahydro-1,2,3,3a,4,5,6,7 triaza-3,6,7a cyclohepta [j k] fluorène.

7. Procédé de préparation des composés (I) dans lesquels $R_2$ est un radical $(C_{1-4})$-alkyle, cyclopropyle, cyclopropylméthyle, benzyle, halogénobenzyle, $(C_{1-4})$- alkyl-benzyle ou $(C_{1-4})$-alcoxy-benzyle, procédé caractérisé en ce que l'on transpose en milieu acide un composé de formule (VII)

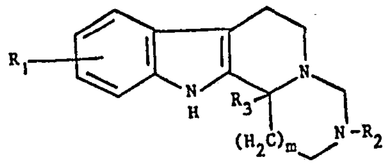

dans laquelle m est 0 ou 1, et $R_1$ et $R_3$ ont les significations de la revendication 1 et $R_2$ a la signification donnée ci-dessus, pour obtenir les composés (I) dans lesquels $R_4 = H$, puis si on le désire, on alkyle ou acyle ces derniers pour obtenir les composés (I) dans lesquels $R_4 = (C_{1-4})$-alkyle, acétyle, propanoyle, benzoyle ou butanoyle.

8. Procédé de préparation des composés (I) dans lesquels $R_2$ est H ou un radical acétyle, $(C_{1-4})$-alcoxycarbonyle ou $(C_{1-4})$-alkylaminocarbonyle, procédé caractérisé en ce que l'on débenzyle le composé (I) correspondant dans lequel $R_2$ est benzyle pour obtenir le composé (I) dans lequel $R_2$ est H, puis, si on le désire, on fait réagir le composé (I) ainsi obtenu avec un composé porteur du groupe $R_2 =$ acétyle, $(C_{1-4})$-alcoxycarbonyle ou $(C_{1-4})$-alkylaminocarbonyle (chloroformiate d'alkyle, anhydride d'acide, chlorure d'acide, isocyanate d'alkyle).

9. Médicament caractérisé en ce qu'il contient un composé spécifié dans l'une quelconque des revendications 1 à 6.

10. Composition pharmaceutique caractérisé en ce qu'elle contient un composé spécifié dans l'une quelconque des revendications 1 à 6 en association avec tout excipient approprié.

**Claims for the Contracting States: BE, CH, DE, FR, GB, IT, LU, NL, SE**

1. Derivatives of fluorenes and fluoranthenes, in the form of racemates or of enantiomers, corresponding to the formula (I)

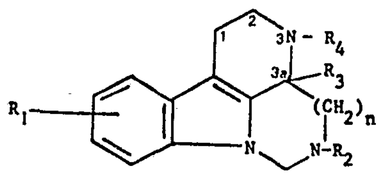

(I)

in which
n is 1 or 2,
$R_1$ is a hydrogen or halogen atom or a $(C_{1-4})$-alkyl or $(C_{1-4})$-alkoxy radical,
$R_2$ is a hydrogen atom, a $(C_{1-4})$-alkyl, cyclopropyl, cyclopropylmethyl, benzyl, halogenobenzyl, $(C_{1-4})$-alkylbenzyl, $(C_{1-4})$-alkoxybenzyl, acetyl, $(C_{1-4})$-alkoxycarbonyl or $(C_{1-4})$-alkylaminocarbonyl radical,
$R_3$ is a hydrogen atom or a $(C_{1-4})$-alkyl radical,
$R_4$ is a hydrogen atom, a $(C_{1-4})$-alkyl, acetyl, propanoyl, benzoyl or butanoyl radical.

2. Derivatives according to claim 1, in which n is 1 or 2, $R_1$ is a fluorine or chlorine atom or a methyl or methoxy radical,
$R_2$ is a hydrogen atom, a $(C_{1-4})$-alkyl, benzyl, cyclopropyl, acetyl, ethoxycarbonyl or methylamino-carbonyl radical,
$R_3$ is a hydrogen atom or a methyl radical.

3. Derivatives according to claim 2, in which n is 2.

4. Derivatives according to claim 1, in which n is 2,
$R_1$ is H or 10-$CH_3O$,
$R_2$ is $CH_3$, cyclopropyl or benzyl,
$R_3$ is H and $R_4$ is $CH_3CO$, $C_6H_5CO$, H, $C_2H_5$ or $C_3H_7$.

5. Derivative according to claim 4, which is the 3 - acetyl - 6 - methyl - 1,2,3,3a,4,5,6,7 octahydro - 3,6,7a - triaza - cyclohepta [jk] fluorene.

6. Derivative according to claim 4, which is 3 - acetyl - 10 - methoxy - 6 - methyl - 1,2,3,3a,4,5,6,7 - octahydro - 3,6,7a - triaza - cyclohepta [jk] fluorene.

7. A process for the preparation of the compounds (I) in which $R_2$ is a $(C_{1-4})$-alkyl, cyclopropyl, cyclopropyl-methyl, benzyl, halogenobenzyl, $(C_{1-4})$ alkylbenzyl, or $(C_{1-4})$-alkoxybenzyl radical, which process comprises acidifying a compound of the formula (VII)

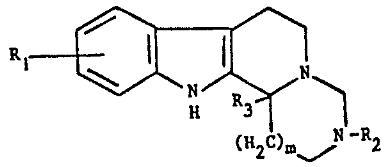

(VII)

in which m is 0 or 1, and $R_1$ and $R_3$ have the meanings given in claim 1 and $R_2$ has the meaning given above, to effect a molecular rearrangement producing compounds (I) in which $R_4$ = H, and thereafter, if desired, alkylating or acylating these compounds to obtain compounds (I) in which $R_4 = (C_{1-4})$-alkyl, acetyl, propanoyl, benzoyl or butanoyl.

8. Process for the preparation of the compounds (I) in which $R_2$ is H or an acetyl, $(C_{1-4})$-alkoxycarbonyl or $(C_{1-4})$-alkylaminocarbonyl, which process comprises debenzylating the corresponding compound (I) in which $R_2$ is benzyl for obtaining the compound (I) in which $R_2$ is H and thereafter, if desired, reacting the compound (I) thus obtained with a compound bearing a $R_2$ group = acetyl, $(C_{1-4})$-alkoxycarbonyl or $(C_{1-4})$-alkylaminocarbonyl (alkyl chloroformate, acid anhydride, acid chloride, alkyl isocyanate).

9. A drug comprising a compound specified in any one of claims 1 to 6.

10. A pharmaceutical composition comprising a compound specified in any one of claims 1 to 6 in association with any suitable excipient.

**Patentansprüche für die Vertragsstaaten: BE, CH, DE, FR, GB, IT, LU, NL, SE.**

1. Fluoren- und Fluoranthenderivative in Form der Racemate oder der Enantiomeren der allgemeinen Formel (I)

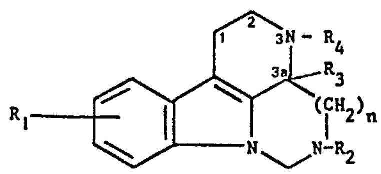

in der
n 1 oder 2,
$R_1$ ein Wasserstoffatom, ein Halogenatom oder eine $(C_{1-4})$-Alkyl- oder $(C_{1-4})$-Alkoxygruppe,
$R_2$ ein Wasserstoffatom, eine $(C_{1-4}$-Alkyl-, Cyclopropyl-, Cyclopropylmethyl-, Benzyl-, Halogenbenzyl-, $(C_{1-4})$-Alkylbenzyl-, $(C_{1-4}$-Alkoxybenzyl-, Acetyl-, $(C_{1-4})$- Alkoxycarbonyl- oder $(C_{1-4})$- Alkylaminocarbonylgruppe,
$R_3$ ein Wasserstoffatom oder eine $(C_{1-4})$-Alkylgruppe,
$R_4$ ein Wasserstoffatom, eine $(C_{1-4})$-Alkyl-, Acetyl-, Propanoyl-, Benzoyl- oder Butanoylgruppe bedeuten.

2. Derivate nach Anspruch 1, dadurch gekennzeichnet, dass n 1 oder 2, $R_1$ ein Fluor- oder Chloratom, eine Methyl- oder Methoxygruppe $R_2$ ein Wasserstoffatom, eine $(C_{1-4})$- Alkyl-, Benzyl, Cyclopropyl-, Acetyl-, Ethoxycarbonyl- oder Methylaminocarbonylgruppe, $R_3$ ein Wasserstoffatom oder eine Methylgruppe, bedeuten.

3. Derivate nach Anspruch 2, dadurch gekennzeichnet, dass n 2 ist.

4. Derivate nach Anspruch 1, dadurch gekennzeichnet dass n für 2, $R_1$ für H oder 10—$CH_3O$, $R_2$ für $CH_3$, Cyclopropyl or Benzyl, $R_3$ für H und $R_4$ für $CH_3CO$, $C_6H_5CO$, H, $C_2H_5$ oder $C_3H_7$ stehen.

5. Derivat nach Anspruch 4, das 3-Acetyl-6-methyl-1,2,3,3a,4,5,6,7-octahydro- 3,6,7a- triazacyclohepta [jk] fluoren ist.

6. Derivat nach Anspruch-4, das 3-Acetyl-10-methoxy-6-methyl- 1,2,3,3a,4,5,6,7- octahydro-3,6,7a-triazacyclohepta [jk] fluoren ist.

7. Verfahren zur Herstellung der Verbindungen (I), in deren $R_2$ eine $(C_{1-4})$- Alkyl-, Cyclopropyl-, Cyclopropylmethyl- Benzyl-, Halogenbenzyl-, $(C_{1-4})$-Alkylbenzyl-oder $(C_{1-4})$-Alkoxybenzylgruppe ist, dadurch gekennzeichnet, dass man in sauerem Medium eine Verbindung (VII)

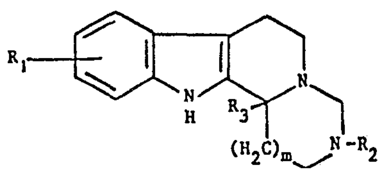

in der
m 0 oder 1 bedeutet, und
$R_1$ und $R_3$ die im Anspruch 1 gegebenen Bedeutungen, und $R_2$ die hieroben gegebene Bedeutung haben, umlagert, um Verbindungen (I), in deren $R_4 = H$, zu herstellen, und gewünschtenfalls man diese letzte Verbindungen (I) alkyliert oder acyliert, um die Verbindungen (I), in deren $R_4 = (C_{1-4})$-Alkyl, Acetyl, Propanyl, Benzoyl oder Butanoyl, zu herstellen.

8. Verfahren zur Herstellung der Verbindungen (I), in deren $R_2$H oder eine Acetyl-, $(C_{1-4})$-Alkoxycarbonyl- oder $(C_{1-4})$-Alkylaminocarbonylgruppe ist, dadurch gekennzeichnet, dass man die entsprechende Verbindung (I), in der $R_2$ Benzyl ist, debenzyliert, um die Verbindung (I), in der $R_2$ H ist, zu herstellen und gewünschtenfalls man die so erhaltene Verbindungen (I) mit einer eine $R_2$ Gruppe = Acetyl, $(C_{1-4})$-Alkoxycarbonyl- oder $(C_{1-4})$-Alkylaminocarbonyl tragenden Verbindung (Alkylchlorformiat, Saüreanhydrid, Saürechlorid, Alkylisocyanat) umsetzt.

9. Arzneimittel, dadurch gekennzeichnet, dass es eine Verbindung nach Ansprüchen 1 to 6 enthalt.

10. Pharmazeutische Zubereitung, dadurch gekennzeichnet dass sie eine Verbindung nach Ansprüchen 1 to 6 enthält.

**Revendications pour l'Etat contractant: AT**

1. Procédé de préparation de dérivés de fluorénes et fluoranthènes, sous la forme de racémates ou d'énantiomères répondant à la formule (I)

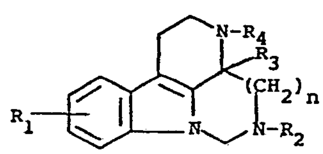

dans laquelle
n est 1 ou 2,
$R_1$ est un atome d'hydrogène ou d'halogène ou un radical $(C_{1-4})$-alkyle ou $(C_{1-4})$-alcoxy,
$R_2$ est un atome d'hydrogène, un radical $(C_{1-4})$-alkyle, cyclopropyle, cyclopropyl-méthyle, benzyle, halogènobenzyle, $(C_{1-4})$-alkyl-benzyle, $(C_{1-4})$-alcoxy-benzyle, acétyle $(C_{1-4})$-alcoxycarbonyle ou $(C_{1-4})$-alkyl-aminocarbonyle,
$R_3$ est un atome d'hydrogène ou un radical $(C_{1-4})$-alkyle,
$R_4$ est un atome d'hydrogène, un radical $(C_{1-4})$-alkyle, acétyle, propanoyle, benzoyle ou butanoyle,
procédé caractérisé en ce que l'on prépare d'abord les composés (I) dans lesquels
$R_2$ est un radical $(C_{1-4})$-alkyle, cyclopropyle, cyclopropylméthyle, benzyle, halogénobenzyle, $(C_{1-4})$-alkyl-benzyle ou $(C_{1-4})$-alcoxy-benzyle, et $R_4$ est H, par transposition en milieu acide d'un composé de formule (VII)

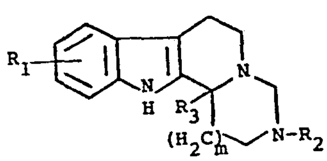

dans laquelle
m est 0 ou 1,
$R_1$ et $R_3$ ont les significations données ci-dessus et $R_2$ est un radical $(C_{1-4})$-alkyle, cyclopropyle, cyclopropylméthyle, benzyle, halogénobenzyle, $(C_{1-4})$ alkyl-benzyle ou $(C_{1-4})$-alcoxy-benzyle, puis on prépare les composés (I) dans lesquels $R_4 = (C_{1-4})$-alkyle, acétyle, propanoyle, benzoyle ou butanoyle, à partir des composés (I) obtenus dans lesquels $R_4 = H$ par alkylation ou acylation, puis on prépare les composés (I) dans lesquels $R_2$ est H par débenzylation des composés (I) correspondants dans lesquels $R_2$ est benzyle et enfin on prépare les composés (I) dans lesquels $R_2$ est un radical acétyle, $(C_{1-4})$-alcoxycarbonyle ou $(C_{1-4})$-alkylaminocarbonyle à partir des composés (I) correspondants dans lesquels $R_2$ est

benzyle par débenzylation puis réaction des composés (I) ainsi obtenus avec un composé porteur du groupe $R_2$ = acétyle, $(C_{1-4})$-alcoxycarbonyle ou $(C_{1-4})$-alkylaminocarbonyle (chloroformiate d-alkyle, anhydride d'acide, chlorure d'acide, isocyanate d'alkyle).

**Claim for the Contracting State: AT**

Process for the preparation of derivatives of fluorenes and fluoroanthenes, in the form of racemates or of enantiomers corresponding to the formula (I)

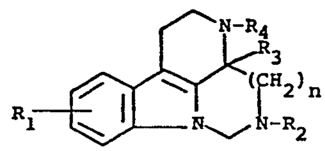

in which
n is 1 or 2
$R_1$ is a hydrogen or a halogen atom or a $(C_{1-4})$-alkyl or $(C_{1-4})$-alkoxy radical,
$R_2$ is a hydrogen atom, a $(C_{1-4})$-alkyl, cyclopropyl, cyclopropylmethyl, benzyl, halogenobenzyl, $(C_{1-4})$-alkylbenzyl, $(C_{1-4})$-alkoxybenzyl, acetyl, $(C_{1-4})$-alkoxycarbonyl or $(C_{1-4})$-alkylaminocarbonyl radical,
$R_3$ is a hydrogen atom or a $(C_{1-4})$-alkyl radical,
$R_4$ is a hydrogen atom, a $(C_{1-4})$-alkyl, acetyl, propanoyl, benzoyl or butanoyl radical,
which process comprises preparing first the compounds (I) in which
$R_2$ is a $(C_{1-4})$-alkyl, cyclopropyl, cyclopropylmethyl, benzyl, halogenbenzyl, $(C_{1-4})$-alkylbenzyl or $(C_{1-4})$-alkoxybenzyl radical, and $R_4$ is H by acidifying a compound of the formula (VII)

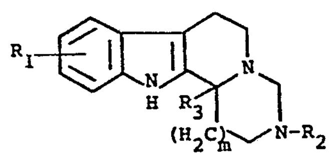

in which
m is 0 or 1,
$R_1$ and $R_3$ have the meanings given above and
$R_2$ is a $(C_{1-4})$-alkyl, cyclopropyl, cyclopropylmethyl, benzyl, halogenobenzyl, $(C_{1-4})$-alkylbenzyl or $(C_{1-4})$-alkoxybenzyl radical, to effect a molecular rearrangement, then preparing compounds (I) in which $R_4$ = $(C_{1-4})$-alkyl, acetyl, propanoyl, benzoyl, or butanoyl, from the obtained compounds (i) in which $R_4$ = H, by alkylating or acylating; thereafter preparing the compounds (I) in which $R_2$ is H by debenzylating the corresponding compounds (I) in which $R_2$ is benzyl and finally preparing the compounds (I) in which $R_2$ is a acetyl, $(C_{1-4})$-alkoxycarbonyl or $(C_{1-4})$-alkylaminocarbonyl radical from the corresponding compounds (I) in which $R_2$ is benzyl by debenzylating and then reacting the compounds (I) thus obtained with a compound bearing a $R_2$ group = acetyl, $(C_{1-4})$-alkoxycarbonyl or $(C_{1-4})$-alkylaminocarbonyl (alkyl chloroformate, acid anhydride, acid chloride, alkyl isocyanate).

**Patentanspruch für die Vertragsstaat: AT**

Verfahren zur Herstellung von Fluroren- und Fluoranthenderivaten in Form der Racemate oder der Enatiomeren der allgemeinen Formel (I)

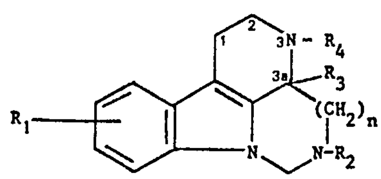

in der
n 1 oder 2,
$R_1$ ein Wasserstoffatom, ein Halogenatom oder eine $(C_{1-4})$-Alkyl- oder $(C_{1-4})$-Alkoxygruppe,
$R_2$ ein Wasserstoffatom, eine $(C_{1-4})$-Alkyl-, Cyclopropyl-, Cyclopropylmethyl-, Benzyl-, Halogenbenzyl-, $(C_{1-4})$-Alkylbenzyl-, $(C_{1-4})$-Alkoxybenzyl-, Acetyl-, $(C_{1-4})$-Alkoxycarbonyl- oder $(C_{1-4})$-Alkylaminocarbonylgruppe,

$R_3$ ein Wasserstoffatom oder eine ($C_{1-4}$-Alkylgruppe, $R_4$ ein Wasserstoffatom, eine ($C_{1-4}$)-Alkyl-, Acetyl-, Propanoyl-, Benzoyl- oder Butanoylgruppe bedeuten, dadurch gekennzeichnet, dass man erst die Verbindungen (I) in deren $R_2$ eine ($C_{1-4}$)- Alkyl-, Cyclopropyl-, Cyclopropylmethyl-, Benzyl-, Halogenbenzyl-, ($C_{1-4}$)-Alkylbenzyl- oder ($C_{1-4}$)-Alkoxybenzylgruppe ist durch Umlagerung in sauerem Medium einer Verbindung (VII)

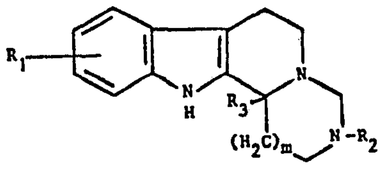

VII

in der m 0 oder 1 bedeutet, und $R_1$ und $R_3$ die oben gegebenen Bedeutungen haben und $R_2$ eine ($C_{1-4}$)- Alkyl-, Cyclopropyl-, Cyclopropylmethyl-, Benzyl-, Halogenbenzyl-, ($C_{1-4}$)-Alkylbenzyl- oder ($C_{1-4}$)-Alkoxybenzylgruppe ist, zubereitet, dann man die Verbindungen (I), in deren $R_4$ = ($C_{1-4}$)-Alkyl, Acetyl, Propanoyl, Benzoyl oder Butanoyl, von den erhaltenen Verbindungen (I), in deren $R_4$ = H, durch Alkylierung oder Acylierung zubereitet, dann man die Verbindungen (I), in deren $R_2$ H ist, durch Debenzylierung der entsprechenden Verbindungen (I) in deren $R_2$ Benzyl ist, zubereitet und zuletzt man die Verbindungen (I), in deren $R_2$ eine Acetyl-, ($C_{1-4}$)-Alkoxycarbonyl-, oder ($C_{1-4}$)-Alkylaminocarbonylgruppe ist, von den enstsprechenden Verbindungen (I), in deren $R_2$ Benzyl ist, durch Debenzylierung und Umsetzung der so erhaltenen Verbindungen (I) mit einer eine $R_2$ Gruppe = Acetyl, ($C_{1-4}$)-Alkoxycarbonyl- oder ($C_{1-4}$)-Alkylaminocarbonyl tragenden Verbindung (Alkylchlorformiat, Säureanhydrid, Säurechlorid, Alkylisocyanat) zubereitet.